# EUROPEAN PATENT SPECIFICATION

(11) **EP 2 341 937 B1**
(45) Date of publication and mention of the grant of the patent: **17.12.2014**
(21) Application number: 09782400.7
(22) Date of filing: 31.08.2009
(51) Int. Cl.: A61K 39/395, A61K 31/519, C07K 16/28, A61P 37/06, C07K 16/46, C12N 15/13

(54) **COMPOSITION FOR TREATING DISEASE**
ZUSAMMENSETZUNG ZUR BEHANDLUNG EINER ERKRANKUNG
COMPOSITION DESTINÉE AU TRAITEMENT DE MALADIES

(30) Priority: 29.09.2008 GB 0817810; 29.09.2008 GB 0817811; 29.09.2008 GB 0817809; 10.03.2009 WO PCT/EP2009/052811; 10.03.2009 WO PCT/EP2009/052809; 10.03.2009 WO PCT/EP2009/052810
(43) Date of publication of application: 13.07.2011
(73) Proprietor: Biotest AG, 63303 Dreieich (DE)
(72) Inventor: OSTERROTH, Frank, 63128 Dietzenbach (DE); AIGNER, Silke, 67227 Frankenthal (DE); UHEREK, Christoph, 63500 Seligenstadt (DE); WARTENBERG-DEMAND, Andrea, 34637 Schrecksbach (DE); RUDNEV, Anatoly, 63303 Dreiech (DE); SOLDAN, Michael, 35216 Biedenkopf (DE); BRUECHER, Christoph, 65760 Eschborn (DE); DAELKEN, Benjamin, 60433 Frankfurt am Main (DE); ZUBER, Chantal, 60528 Frankfurt am Main (DE); SCHULZ, Gregor, 79224 Umkirch (DE); CZELOTH, Niklas, 63303 Dreieich (DE)
(74) Representative: Daniels, Jeffrey Nicholas
(86) International application number: PCT/EP2009/061210
(87) International publication number: WO 2010/034590

(56) References cited:
- WO-A1-2004/083247
- LUGGEN M E ET AL: "Results of a phase II, double-blind, randomized study of a nondepleting anti-CD4 monoclonal antibody (Clenoliximab) given in combination with methotrexate (MTX) in patients with moderate to severe rheumatoid arthritis (RA)" ANNALS OF THE RHEUMATIC DISEASES, BRITISH MEDICAL ASSOCIATION, LONDON, GB, vol. 62, no. Suppl. 1, 1 July 2003 (2003-07-01), page 99, XP008113414 ISSN: 0003-4967
- SOUNDARARAJAN D ET AL: "Clinical and immunological effects of a primatized anti CD4 antibody used concomitantly with methotrexate in rheumatoid arthritis" JOURNAL OF ALLERGY AND CLINICAL IMMUNOLOGY, MOSBY - YEARLY BOOK, INC, US, vol. 99, no. 1 part 2, 1 January 1997 (1997-01-01), page S193, XP008113383 ISSN: 0091-6749
- EHRENSTEIN MICHAEL R ET AL: "Compromised function of regulatory T cells in rheumatoid arthritis and reversal by anti-TNFalpha therapy" JOURNAL OF EXPERIMENTAL MEDICINE, ROCKEFELLER UNIVERSITY PRESS, JP, vol. 200, no. 3, 2 August 2004 (2004-08-02), pages 277-285, XP002486681 ISSN: 0022-1007
- RACADOT E ET AL: "IMMUNOLOGICAL FOLLOW-UP OF 17 PATIENTS WITH RHEUMATOID ARTHRITIS TREATED IN VIVO WITH AN ANTI-T CD4+ MONOCLONAL ANTIBODY (B-F5)" CLINICAL AND EXPERIMENTAL RHEUMATOLOGY, PACINI, PISA, IT, vol. 10, no. 4, 1 January 1992 (1992-01-01), pages 365-374, XP008022443 ISSN: 0392-856X
- WENDLING D ET AL: "TREATMENT OF RHEUMATOID ARTHRITIS WITH ANTI CD4 MONOCLONAL ANTIBODY. OPEN STUDY OF 25 PATIENTS WITH THE B-F5 CLONE" CLINICAL RHEUMATOLOGY, ACTA MEDICA BELGICA, BRUXELLES, BE, vol. 11, no. 4, 1 December 1992 (1992-12-01), pages 542-547, XP008022444 ISSN: 0770-3198
- BAYRY ET AL: "Rescuing CD4+CD25+ regulatory T-cell functions in rheumatoid arthritis by cytokine-targeted monoclonal antibody therapy" DRUG DISCOVERY TODAY, ELSEVIER, RAHWAY, NJ, US, vol. 12, no. 13-14, 19 July 2007 (2007-07-19), pages 548-552, XP022164789 ISSN: 1359-6446
- YI ET AL: "The effects of antibody treatment on regulatory CD4<+>CD25<+> T cells" TRANSPLANT IMMUNOLOGY, ELSEVIER, vol. 19, no. 1, 28 December 2007 (2007-12-28), pages 37-44, XP022533978 ISSN: 0966-3274
- Anonymous: "Further Interim Analyses Confirm Good Clinical Efficacy of the BT-061 Monoclonal Antibody" Press Release Biotest at EvaluatePharma 7 July 2009 (2009-07-07), XP002553086 Retrieved from the Internet: URL:http://www.evaluatepharma.com/Universa l/View.aspx?type=Story&id=189929> [retrieved on 2009-10-14]
- Christian Becker ET AL: "CD4-mediated functional activation of human CD4+CD25+ regulatory T cells", Eur. J. Immunol., vol. 37, 1 January 2007 (2007-01-01), pages 1217-1223, XP55042509, DOI: 10.1002/eji.200636480]
- VALENCIA XAVIER ET AL: "TNF downmodulates the function of human CD4(+)CD25(hi) T-regulatory cells", BLOOD, AMERICAN SOCIETY OF HEMATOLOGY, US, vol. 108, no. 1, 1 July 2006 (2006-07-01), pages 253-261, XP002419715, ISSN: 0006-4971, DOI: 10.1182/BLOOD-2005-11-4567

## Description

The present invention is concerned with treatment of rheumatic diseases. The invention involves a highly effective pharmaceutical composition comprising an agent capable of activating CD4+CD25+ regulatory T cells, which is a humanised monoclonal antibody, and the drug methotrexate. The composition and kits of the invention are particularly effective in the treatment of rheumatoid arthritis. The invention envisages pharmaceutical compositions or kits comprising the agent and methotrexate, as well as medical uses employing the composition and kits.

Rheumatic diseases are a group of diseases affecting the connective tissue, especially the joints and related structures, and are characterized by inflammation, degeneration or metabolic derangement. Examples of rheumatic diseases are rheumatoid arthritis, psoriatic arthritis, juvenile rheumatoid arthritis and ankylosing spondylitis.

Rheumatoid arthritis is an autoimmune disease which causes chronic inflammation of joints and surrounding tissues, and can also affect other tissues and body organs. The disease occurs when T cells, which are normally tolerant with regard to autologous tissue, recognise and react to 'self molecules, that is, molecules produced by the cells of the host. Activation of 'autoreactive' T cells by presentation of autoantigens processed by antigen presenting cells (APC) leads to their clonal expansion and migration to the specific tissues, where they induce inflammation and tissue destruction.

There are a large range of treatments for rheumatoid arthritis which are currently available, including first line drugs for controlling pain and inflammation classified as non-steroidal anti-inflammatory drugs (NSAIDs), e.g., aspirin, ibuprofen, naproxen, etc. Secondary treatment of arthritis includes corticosteroids (e.g. prednisone and dexamethasone), which are synthetic versions of the body's cortisone hormone, slow acting antirheumatic drugs (SAARDs) or disease-modifying anti-rheumatic drugs (DMARDs), e.g., hydroxycloroquine, sulfasalazine, methotrexate, penicillinamine, cyclophosphamide, gold salts, azothipoprine, leflunomide, etc.

Many patients with newly diagnosed RA are started with a DMARD, such as methotrexate (MTX). MTX (4-amino-N10-methylpteroyl glutamic acid) is an analogue of folic acid, which is known to hamper the production of a form of folic acid important for actively growing cells such as those found in the skin, blood, gastrointestinal tissues and those involved in the immune system. It is not entirely clear how MTX decreases the severity of RA, however, it is thought to play a role in anti-inflammatory action and a variety of pharmacological mechanisms for its action have been proposed, including inhibition of purine synthesis, promotion of adenosine release, inhibition of production of proinflammatory cytokines, and modulation of inflammation (Swierkot et al., 2006, Pharmacological Reports 2006; 58: 473-492). MTX is also known to inhibit, for example, the activity of an enzyme called dihydrofolate reductase (DHFR), and also interferes with several other enzymes.

Another group of drugs for the treatment of RA are called biological-response modifiers (BRMs), which includes monoclonal antibodies. Examples of these are antagonists to tumour necrosis factor-alpha (TNF-alpha), such as adalimumab, infliximab, and etanercept, which work through binding to the TNF-alpha receptor or directly binding to the TNF-alpha protein itself. Several TNF-alpha inhibitors have been approved by the FDA for treatment of rheumatoid arthritis, including adalimumab (Humira®), Infliximab (Remicade®) and Etanercept (Enbrel®). TNF-alpha represents a key mediator in rheumatoid arthritis, and is mainly produced by activated macrophages within the synovium of RA patients. Acting as a pro-inflammatory cytokine, TNF alpha is abundantly present in the synovial tissue of RA patients. It induces the production and release of chemokines that attract leukocytes from the blood into the inflamed tissue (Tracey et al., 2008, Pharmacol Ther. 2008;117(2):244-79). Beside the mediation of synovial inflammation, TNF alpha is involved in joint destruction and cartilage degradation. Additionally, it is capable of inhibiting the suppressive activity of CD4+CD25+ regulatory T-cells (Andersson et al., 2008, Scand J Immunol. 2008; 68(1):103-11).

In some cases RA patients are treated with a combination of the drugs discussed above. In particular, the DMARDs are frequently used as a first treatment. However, it can be desirable in patients where disease control is not achieved, to use them in combination with treatments that have been more recently approved, such as biological agents e.g. TNF-alpha antagonists. It has been reported that a combination of MTX with some monoclonal antibodies (etanercept, infliximab, adalimumab and anakirina) leads to a better therapeutic efficacy as compared to MTX therapy alone (Swierkot et al., Pharmacological Reports 2006; 58: 473-492). However, MTX exerts a variety of pharmacological actions and its clinical effects can be attributed to multiple targets (Wessels et al., 2008, Rheumatology (Oxford) 2008; 47(3):249-55). Accordingly, it cannot readily be predicted how MTX will affect the therapeutic activity, and therefore the efficacy, of a drug which is effective as a single agent.

Despite the range of currently available drugs, not all patients respond well to the above treatments and there are a number of adverse side effects. For example, TNF-alpha treatment down regulates the immune system making the treated patients more susceptible to infections and disease. Accordingly, there is still a need for alternative therapies to be developed.

Luggen et al., disclose the results of a phase II double-blind, randomized study of a nondepleting anti-CD4 monoclonal antibody (clenoliximab) given in combination with methotrexate (MTX) in patients with moderate to severe rheumatoid arthritis (Annuals of the Rheumatic Diseases, Vol. 62, No. Suppl. 1, 1 July 2003, page 99).

It is generally agreed that CD4⁺ T cells play a major part in initiating and maintaining autoimmunity. Accordingly, it has been proposed to use mAbs against CD4⁺ T cells surface molecules, and in particular anti-CD4 mAbs, as immunosuppressive agents in the treatment of diseases such as rheumatoid arthritis.

One example under further investigation is the anti-CD4 B-F5 antibody (murine IgG1 antihuman CD4), which has been tested in different autoimmune diseases. In rheumatoid arthritis patients, the results observed in a placebo controlled trial with a daily dose of B-F5 did not indicate a significant improvement (Wendling et al. J Rheumatol; 25(8):1457-61, 1998). However, in WO 2004/083247, a humanized B-F5 (hereinafter referred to as hB-F5 or BT061) antibody having similar CD4 binding properties to the parent mouse B-F5 was developed. A preliminary evaluation of the effect of the humanized version of the mouse B-F5 antibody in patients also receiving the non-steroidal anti-inflammatory drug Diclophenac provided an indication of an effective immunosuppression, reflected by a positive clinical effect in the patients when used in a 10 day treatment.

The study was also described by Wijdenes et al., in an abstract and poster presented at the EULAR conference, June 2005. They described the treatment of 11 patients suffering from rheumatoid arthritis with 5 intravenous infusions of 5 mg hB-F5 every other day with concomitant treatment with 150 mg Diclophenac (Wijdenes et al., Abstract and poster, EULAR conference, June 2005).

In WO 2004/083247 it was noted that the antibody was able to activate a particular subset of CD4+ T cells, namely CD4+CD25+ regulatory T cells (Tregs). These cells constitute 5-10% of peripheral CD4+ T cells and, once stimulated, are competent to suppress the response of CD4+ T cells and CD8+ T cells as well as inhibit B-cell activation and clonal expansion. Thus these cells represent an important level of control in the immune system. In particular, CD4⁺CD25⁺Treg cells are involved in maintaining immune homeostasis in the periphery, and regulating autoimmunity and pathogenic immune responses.

The treatment of rheumatoid arthritis via a mechanism which activates CD4+CD25+ regulatory T cells represents an important avenue of research, and the use of the hB-F5 agent in phase II clinical trials on patients suffering from rheumatoid arthritis is discussed in International Patent Application publication numbers WO 2009/121690, WO 2009/124815 and WO 2009/112502.

However, it cannot readily be predicted whether any new treatment can be successfully combined with the current treatments to give a beneficial therapeutic effect. As mentioned above, this is particularly the case with MTX.

Several studies have reported findings which suggest that MTX has a negative effect on regulatory T cells and therefore is likely to prevent MTX being used in a combination therapy with an agent which relies on activation of CD4+CD25+ regulatory T cells for its therapeutic mechanism. Wascher et al., (Clin Invest. 1994 Jul;72(7):535-40) and Herman et al., (Inflamm Res. 2005 Jul;54(7):273-80) report findings that suggest that MTX may reduce the number of available T lymphocytes. Wascher et al., reported that administration of high dose MTX given intravenously at 12 weeks significantly (P < 0.01) reduced total peripheral blood lymphocytes and led to a pronounced redistribution of lymphocyte subsets with a preferred reductive effect on B-lymphocytes (P < 0.005) and T lymphocytes (P < 0.05). Herman et al., reported an apoptosis inducing effect of MTX in T lymphocytes in vitro, at concentrations reflecting a low dose therapy of RA patients (7.5 mg).

Further, an in vitro study conducted by Porter et al., (Cell Therapy and Islet Transplantation. 2006. 83(1); 23-29) reported an influence of MTX on the viability of regulatory T-cells. At in vitro concentrations of 50 nM (highest concentration analyzed) the suppressive activity of Treg cells was significantly reduced from 94% to 88% (p<0.05). This suggests that the presence of MTX may inhibit Treg suppression.

Still further, Yamaguchi et al., (Immunity. 2007 Jul;27(1):145-59) reported that natural Treg cells constitutively expressed high amounts of folate receptor 4 (FR4). Since MTX is a folate analogue, it is suggested that MTX may also be taken up by Tregs cells. Such an uptake is likely to result in interference with metabolism within this cell population.

Moreover, it is known that the therapeutic activity of many antibodies is influenced by Fc receptors on Fc receptor expressing cells. Some antibodies even need to bind to Fc receptors on Fc receptor expressing cells to be active. However, MTX therapy is known to result in a decrease of Fc gamma R1 expression on monocytes (Wijngaarden et al., Arthritis Rheum. 2004 Dec;50(12):3878-87, and Wijngaarden et al., Rheumatology (Oxford). 2005 Jun;44(6):729-34) in vivo. The reductive influence of MTX on Fc receptor expression on monocytes has also been demonstrated in patients that were treated with MTX and the therapeutic anti-TNF-alpha antibody Infliximab (Wijngaarden et al., Clin Exp Rheumatol. 2008 Jan-Feb;26(1):89-95). As a result, it is generally considered that MTX negatively influences the activity of Fc receptor binding antibodies. Accordingly, it is expected that MTX will negatively influence the capacity of an antibody to activate Tregs.

Given the above, it is expected that MTX will have a negative impact on the therapeutic capacity of an agent which works via the activation of CD4+CD25+ regulatory T cells, such as hB-F5. As such, it can be seen that the outcome of the combinative treatment approaches cannot be predicted.

Having regard to the above described prior art, it is the aim of the present invention to develop further and improved pharmaceutical compositions for the treatment of rheumatoid arthritis.

Accordingly, in a first aspect the present invention provides a kit comprising separately an agent capable of activating CD4+CD25+ regulatory T cells and methotrexate, wherein the agent is a humanized anti-CD4 antibody which comprises an H chain V domain and an L chain V domain, and wherein: (a) the H chain V domain comprises the sequences DCRMY, VISVKSENYGANYAESVRG and SYYRYDVGAWFAY, and the L chain V domain comprises the sequences RASKSVSTSGYSYIY, LASILES and QHSRELPWT; and/or (b) the H chain V domain has at least 80% sequence identity with SEQ ID No: 1 and the L chain V domain has at least 80% sequence identity with SEQ ID No: 2.

The present inventors have unexpectedly found that a combination of an agent capable of activating CD4+CD25+ regulatory T cells with methotrexate has a therapeutic effect, and is surprisingly advantageous in relation to the reduced number of antibody-related side effects. The combination is also surprisingly advantageous in relation to the speed at which a high level therapeutic effect is reached.

Accordingly, the present invention also provides an agent capable of activating CD4+CD25+ regulatory T cells and methotrexate as a combined preparation for simultaneous, separate or sequential use in medicine, wherein the agent is a humanized anti-CD4 antibody which comprises an H chain V domain and an L chain V domain, and wherein: (a) the H chain V domain comprises the sequences DCRMY, VISVKSENYGANYAESVRG and SYYRYDVGAWFAY, and the L chain V domain comprises the sequences RASKSVSTSGYSYIY, LASILES and QHSRELPWT; and/or (b) the H chain V domain has at least 80% sequence identity with SEQ ID No: 1 and the L chain V domain has at least 80% sequence identity with SEQ ID No: 2.

The present invention also provides an agent capable of activating CD4+CD25+ regulatory T cells for use in the treatment of a rheumatic disease, wherein the agent is for simultaneous, separate or sequential use with methotrexate as a combination therapy, wherein the agent is a humanized anti-CD4 antibody which comprises an H chain V domain and an L chain V domain, and wherein: (a) the H chain V domain comprises the sequences DCRMY, VISVKSENYGANYAESVRG and SYYRYDVGAWFAY, and the L chain V domain comprises the sequences RASKSVSTSGYSYIY, LASILES and QHSRELPWT; and/or (b) the H chain V domain has at least 80% sequence identity with SEQ ID No: 1 and the L chain V domain has at least 80% sequence identity with SEQ ID No: 2.

In particular, the agent for the use specified in the above paragraph can be for use in the treatment of a rheumatic disease in a patient who is a non-responder to treatment with a disease modifying anti-rheumatic drug (DMARD), preferably wherein the DMARD is methotrexate.

Further, the present invention provides an agent capable of activating CD4+CD25+ regulatory T cells for use in the treatment of a rheumatic disease in a patient, wherein the patient is undergoing methotrexate treatment, wherein the agent is a humanized anti-CD4 antibody which comprises an H chain V domain and an L chain V domain, and wherein: (a) the H chain V domain comprises the sequences DCRMY, VISVKSENYGANYAESVRG and SYYRYDVGAWFAY, and the L chain V domain comprises the sequences RASKSVSTSGYSYIY, LASILES and QHSRELPWT; and/or (b) the H chain V domain has at least 80% sequence identity with SEQ ID No: 1 and the L chain V domain has at least 80% sequence identity with SEQ ID No: 2.

Alternatively, the present invention provides a composition comprising methotrexate for use in the treatment of a rheumatic disease in a patient, wherein the patient is undergoing treatment with an agent capable of activating CD4+CD25+ regulatory T cells, wherein the agent is a humanized anti-CD4 antibody which comprises an H chain V domain and an L chain V domain, and wherein: (a) the H chain V domain comprises the sequences DCRMY, VISVKSENYGANYAESVRG and SYYRYDVGAWFAY, and the L chain V domain comprises the sequences RASKSVSTSGYSYIY, LASILES and QHSRELPWT; and/or (b) the H chain V domain has at least 80% sequence identity with SEQ ID No: 1 and the L chain V domain has at least 80% sequence identity with SEQ ID No: 2.

In a still further aspect the present invention provides a method comprising preparing a kit comprising the agent and the methotrexate according to the first aspect of the invention.

The invention will be illustrated by way of example only, with reference to the following Figures, in which:
Figure 1 shows the results of an in vitro proliferation assay conducted with CD4+CD25+ regulatory T cells taken from two donors (Exp.1 and Exp. 2) in Example 1.
Figures 2A and 2B show graphs of the % of patients achieving at least an ACR 20 score over the course of the clinical trial described in Example 2 as compared with patients in the most effective dose groups reported in phase III trials published by Keystone et al., (Arthritis Rheum. 2004 May;50(5): 1400-11) and (Ann Rheum Dis. 2009 Jun;68(6):789-96). The graph in Figure 2B is placebo-corrected.
Figures 3A and 3B show graphs of the % of patients achieving at least an ACR 50 score over the course of the clinical trial described in Example 2 as compared with patients in the most effective dose groups reported in phase III trials published by Keystone et al., (Arthritis Rheum. 2004 May;50(5): 1400-11) and (Ann Rheum Dis. 2009 Jun;68(6):789-96). The graph in Figure 3B is placebo-corrected.
Figures 4A and 4B show graphs of the % of patients achieving at least an ACR 70 score over the course of the clinical trial described in Example 2 as compared with patients in the most effective dose groups reported in phase III trials published by Keystone et al., (Arthritis Rheum. 2004 May;50(5):1400-11) and (Ann Rheum Dis. 2009 Jun;68(6):789-96). The graph in Figure 4B is placebo-corrected.

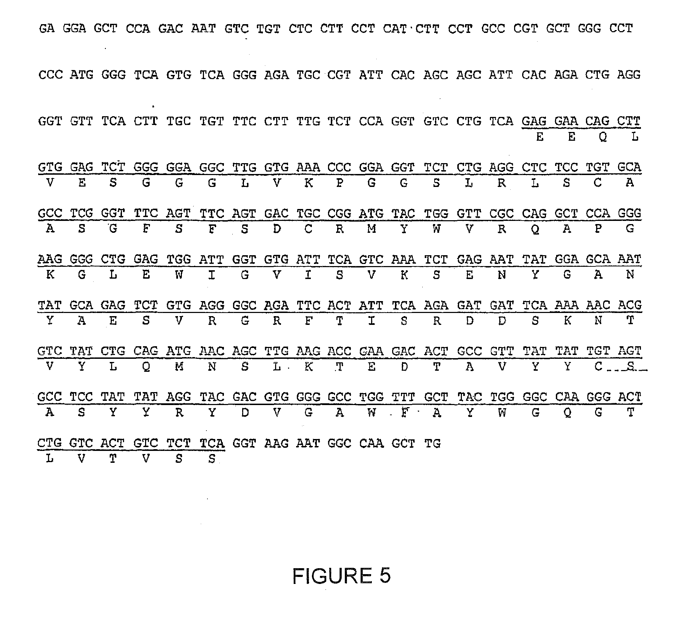

Figure 5 shows the nucleotide sequence (SEQ ID No: 3) of a fragment of the plasmid encoding the V_{H} region of humanized B-F5. The sequence encoding the V region is underlined and the corresponding polypeptide sequence (SEQ ID No: 15) is indicated below the nucleotide sequence;

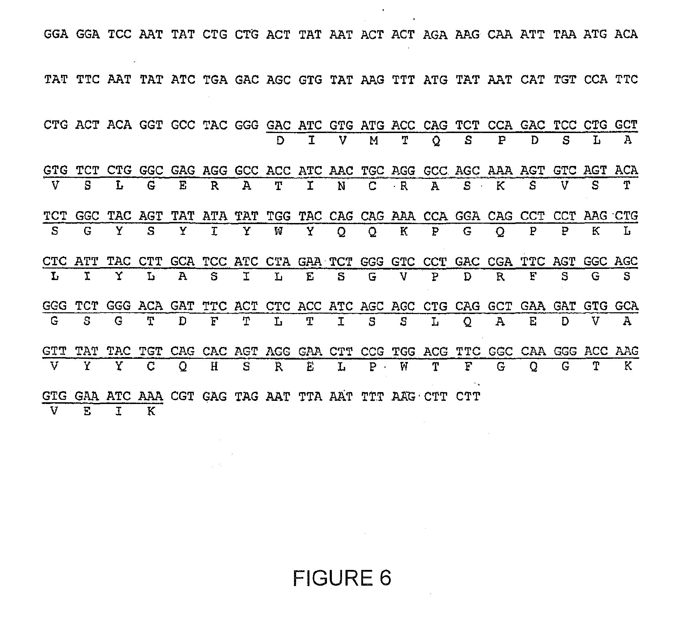

Figure 6 shows the nucleotide sequence (SEQ ID No: 4) of a fragment of the plasmid encoding the V_{K} regions of humanized B-F5. The sequence encoding the V region is underlined and the corresponding polypeptide sequence (SEQ ID No: 2) is indicated below the nucleotide sequence;
Figure 7 shows the alignment of the polypeptide sequences of murine B-F5 V_{K} (SEQ ID No: 6), FK-001 (SEQ ID Nos: 7, 8 9 and 10), L4L (SEQ ID No: 16), and L4M (SEQ ID No: 2) in the design of the humanised form of B-F5 (i.e. BT061).
Figure 8 shows the alignment of the polypeptide sequences of murine B-F5 V_{H} (SEQ ID No: 5), M26 (SEQ ID Nos: 11, 12, 13 and 14), H37L (SEQ ID No: 1), and H37V (SEQ ID No: 15) in the design of the humanised form of B-F5.

The present description provides a pharmaceutical composition comprising an agent capable of activating CD4+CD25+ regulatory T cells and methotrexate. The present invention provides a kit comprising separately an agent capable of activating CD4+CD25+ regulatory T cells and methotrexate, as defined above.

The agent capable of activating CD4+CD25+ regulatory T cells and the methotrexate may be in a single formulation or in separate formulations. The formulations may consist of the agent and/or the methotrexate. Alternatively, the formulations may comprise the agent and/or the methotrexate, and further comprise pharmaceutically acceptable components, such as carriers or excipients.

In one aspect the agent and/or methotrexate are adapted for parenteral administration, preferably intramuscular, intravenous or subcutaneous administration. It is most preferred that the agent and/or methotrexate are suitable for subcutaneous administration.

In one embodiment of this aspect the agent and/or methotrexate are adapted for intravenous administration and are provided in a dosage volume of 0.5 to 500 ml or in a form for dilution to the dosage volume of 0.5 to 500 ml. In an alternative embodiment the composition is suitable for subcutaneous or intramuscular administration and is provided in a dosage volume of 0.1 to 3 ml. Alternatively, the agent and/or methotrexate are suitable for providing a dosage volume of 0.5 to 1.5 ml or 15 to 25 ml.

In an alternative aspect the methotrexate is adapted for oral administration and may be in tablet form.

In further aspects the composition or kit may be suitable for use as a single dose or suitable for use as part of a plurality of doses, in particular, where the dose is to be administered weekly, once every two weeks, once every four weeks, once every six weeks or once every eight weeks.

In one embodiment of this aspect the kit comprises a plurality of separate doses of the agent and the methotrexate. In a further embodiment, a dosage pack is provided comprising a plurality of separately packaged doses of the pharmaceutical composition.

In one specific embodiment the agent, optionally with the methotrexate, is suitable for subcutaneous administration and is provided in a ready for administration form which does not require dilution so that they can be easily administered by non-medical personnel.

The agents that are suitable for use are those which are capable of activating CD4+CD25+ regulatory T cells. The agent may be a polypeptide, a protein or an antibody. Where the agent is an antibody it may be a monoclonal antibody. Preferably the antibody is a monoclonal anti-CD4 antibody. The antibody may also preferably be an IgG1 antibody and may be an unmodified IgG1 antibody.

The antibodies which are most suitable for use are humanized anti-CD4 antibodies, or fragments or derivatives thereof, which are capable of activating CD4+CD25+ regulatory T cells. Examples of antibodies which are capable of activating CD4+CD25+ regulatory T cells are discussed in Becker et al., (European Journal of Immunology (2007), Vol. 37: pages 1217-1223) and are described in WO 2004/083247.

Generally the antibody used comprises one or more variable domains which are capable of binding to CD4. The antibody may comprise a human constant region (Fc). This constant region can be selected among constant domains from any class of immunoglobulins, including IgM, IgG, IgD, IgA and IgE, and any isotype, including IgG1, IgG2, IgG3 and IgG4. Preferred constant regions are selected among constant domains of IgG, in particular IgG1.

The present description also includes any fragment of the antibody. Fragments preferably comprise the antigen binding or V regions of the antibody, and are in particular Fab, Fab', F(ab)'₂, Fv and scFv fragments.

In a particularly preferred aspect the antibody is a humanized anti-CD4 antibody or fragment or derivative thereof derived from the mouse monoclonal anti-CD4 antibody B-F5. The antibody may be a humanized anti-CD4 antibody which comprises a sequence comprising the complementarity-determining regions (CDRs) of the mouse monoclonal antibody B-F5, optionally with variations in the sequence that do not substantially affect the antibody specificity and/or affinity thereof.

Examples of antibodies are provided in WO 2004/083247, in which the production of several humanised versions of the mouse B-F5 antibody is disclosed. In particular, WO 2004/083247 discloses the production of a humanised antibody BT061 (hB-F5) having V domains defined by the following polypeptide sequences:
- H chain V domain: EEQLVESGGGLVKPGGSLRLSCAASGFSFSDCRMYWLRQA PGKGLEWIGVISVKSENYGANYAESVRGRFTISRDDSKNTVYLQMNSLKTEDTAVY YCSAS YYRYDVGAWFAYWGQGTLVTVSS (SEQ ID NO: 1)
- L chain V domain:

Derivatives of this antibody are also suitable for the uses described herein. Derivatives include those with V domains defined by polypeptide sequences having at least 80%, preferably at least 90%, most preferably at least 95% sequence identity with SEQ ID NO: 1 or SEQ ID NO: 2.

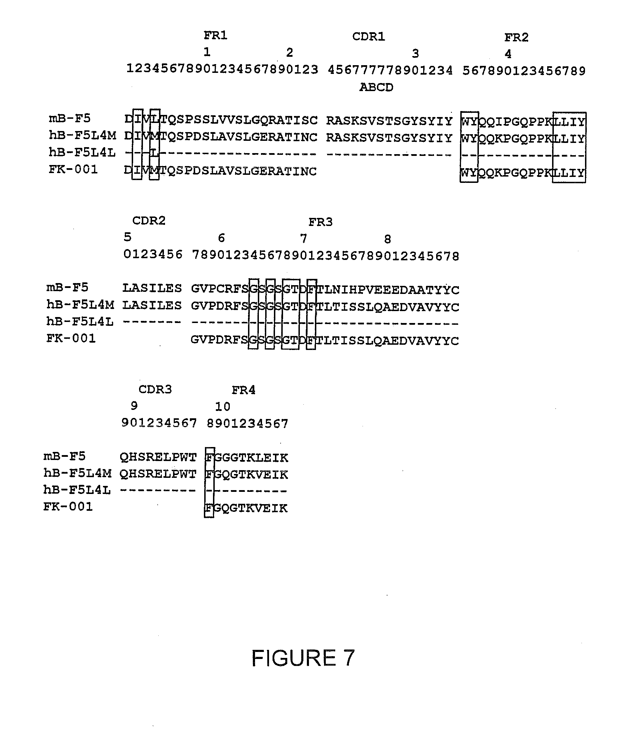

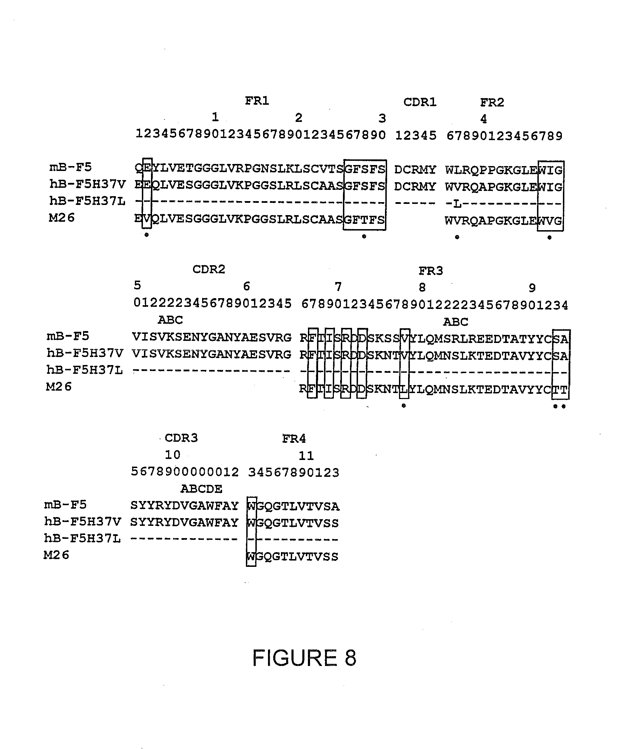

Particularly preferred antibodies are those which comprise the complementarity-determining regions (CDRs) of the mouse B-F5 mAb, and retain the ability of hB-F5 to activate CD4+ CD25+ regulatory T cells. The location of the CDRs within the V_{H} and V_{K} domains is shown in Figures 7 and 8. Such antibodies can optionally have variations in the sequence of the CDRs that do not substantially affect the specificity and/or affinity of binding.

Generally, the antibody used further comprises a human constant region (Fc). This constant region can be selected from among the constant domains indicated above.

The present description also includes any fragment of the hB-F5 antibody or derivative thereof comprising the V regions thereof. This comprises in particular Fab, Fab', F(ab)'₂, Fv and scFv fragments.

In order to prepare the hB-F5 antibody a polynucleotide encoding the V domain of the H chain or of the L chain of a BT061 antibody may be fused with a polynucleotide coding for the constant region of a human H or L chain. For the purpose of expressing the complete H and L chains obtained in this way a sequence coding a signal peptide allowing the secretion of the protein can also be added.

The polynucleotide as described above is linked within an expression vector to appropriate control sequences allowing the regulation of its transcription and translation in a chosen host cell. These recombinant DNA constructs can be obtained and introduced in host cells by the well-known techniques of recombinant DNA and genetic engineering.

Useful host-cells can be prokaryotic or eukaryotic cells. Among suitable eukaryotic cells, one will mention, by way of example, plant cells, cells of yeasts such as *Saccharomyces,* cells of insects such as *Drosophila*, or *Spodoptera*, and mammal cells such as HeLa, CHO, 3T3, C127, BHK, COS, etc.

The BT061 (hB-F5) antibody utilised in the invention can be obtained by culturing a host cell containing an expression vector comprising a nucleic acid sequence encoding said antibody, under conditions suitable for the expression thereof, and recovering said antibody from the host cell culture.

A further aspect is a method comprising preparing a kit or a pharmaceutical composition comprising the agent and the methotrexate.

As indicated above, the present description further provides medical uses and methods of treatment of patients suffering from, or susceptible to rheumatic diseases. In particular, in one aspect a method of treating a rheumatic disease in a patient is provided comprising a step (a) of administering an agent capable of activating CD4+CD25+ regulatory T cells and a step (b) of administering methotrexate, wherein step (a) and step (b) can be conducted simultaneously, separately or sequentially and in either order. In one embodiment of this aspect step (a) and step (b) are conducted on the same day. In an alternative embodiment of this aspect step (a) and step (b) are conducted within the same week.

In alternative aspects, the present description provides a method of treating a rheumatic disease in a patient undergoing methotrexate treatment comprising a step of administering an agent capable of activating CD4+CD25+ regulatory T cells, and a method of treating a rheumatic disease in a patient undergoing treatment with an agent capable of activating CD4+CD25+ regulatory T cells comprising a step of administering methotrexate.

Rheumatic diseases are defined as diseases affecting the connective tissue, especially the joints and related structures, in particular being characterized by inflammation, degeneration or metabolic derangement. In a preferred aspect of the invention the rheumatic disease is rheumatoid arthritis, psoriatic arthritis, juvenile rheumatoid arthritis or ankylosing spondylitis.

The treatment of rheumatoid arthritis is preferred. With rheumatoid arthritis clinical efficacy of treatment may be assessed using ACR scoring.

ACR scoring is a method of assessment of rheumatoid arthritis exhibited by a treated patient set out by the American College of Rheumatology (ACR) and works through the measurement of a core set of parameters (Felson et al., Arthritis & Rheumatism, 1995, 38(6), 727-735). This system defines a value of ACR 20 as an at least 20% improvement in tender and swollen joint counts and at least 20% improvement in 3 of the 5 remaining ACR core set measures: patient and physician global assessments, pain, disability, and an acute phase reactant, such as C-reactive protein (CRP) or Erythrocyte Sedimentation Rate (ESR). Similarly, ACR 50 and ACR 70 scores define an at least 50% and an at least 70% improvement, respectively.

In a further aspect the treatment may be administered to a patient who is a non-responder to treatment with a disease-modifying anti-rheumatic drug (DMARD). A non-responder is a patient who shows an inadequate response to treatment with a DMARD. In particular, a patient shows an inadequate response if he/she has continuing clinically active rheumatoid arthritis, e.g. if the drug is not achieving ACR20 in the patient or is not achieving an inhibition of progression of structural damage to the joints or if an initial response to the drug is lost over time during treatment.

Examples of DMARDs are e.g., hydroxycloroquine, sulfasalazine, methotrexate, penicillinamine, cyclophosphamide, gold salts, azothipoprine, leflunomide, etc.

As indicated above, the agent and the methotrexate may be administered to the patient in any suitable manner. In particular, they may be administered parenterally, for example by intravenous, intramuscular or subcutaneous injection. For administration of the agent, intravenous or subcutaneous administration is particularly preferred. Further, methotrexate may be administered orally.

The volume in which the agent and/or methotrexate are dosed will vary depending on the method of administration. Where the dose is to be administered by intravenous infusion the dosage volume may be from 0.1 or 0.5 ml up to 500 ml, preferably between 15 and 25 ml, and typically about 20 ml. Where the dose is to be administered by subcutaneous or intramuscular injection, the dosage volume may be between 0.1 to 3 ml, preferably between 0.5 and 1.5 ml, and typically about 1 ml.

The frequency of administration is not especially limited, provided that it does not interfere with the effectiveness of the treatment. Treatment may comprise a single dose or a plurality of doses. It is preferred that the plurality of doses are administered on at least the following bases: weekly, every two weeks, every 4 weeks, every 6 weeks, every 12 weeks, every 24 weeks, every calendar month, every 6 calendar months or yearly. Thus, the doses may be separated by at least one week, or by at least two weeks, at least one month or by at least 3 months or by at least 6 months or by at least one year (meaning that the doses are taken every week, every two weeks, or every month or every 6 months or every year). It is particularly preferred that the doses are administered at least every two to three weeks.

The length of treatment is not especially limited, and, as is typical in the treatment of autoimmune diseases, the treatment proceeds indefinitely, or until symptoms are reduced to a manageable level for the patient. Generally the treatment is administered to the subject for at least 1 month.

It will be understood that the agent and the methotrexate are to be administered in therapeutically effective amounts, i.e. in amounts which are effective for ameliorating, preventing or treating the rheumatic disease.

In particular, where the disease is rheumatoid arthritis, the agent and the methotrexate are preferably administered in an amount that is effective to provide an ACR50 response, more preferably an ACR70 response.

In one aspect the agent is to be administered to a subject in a dose from 0.2 to 10 mg, and more preferably in a dose from 0.2 to 6.25 mg, and most preferably in a dose from 0.2 to 3 mg. These doses are particularly preferred where the dose is administered intravenously.

Where the agent is the humanised antibody BT061 the inventors have surprisingly found that the effective Cmax values of the antibody circulating in the plasma of healthy volunteers 3 hours after the end of intravenous infusion are much lower than expected, as shown in Table 1, below. This is considered to reflect a faster target mediated clearance.

**Table 1 - the expected and effective plasma levels of BT061 after intravenous administration. The range of plasma levels measured in three different volunteers per dose-group is indicated.**

| | **plasma level (µg/ml)** | | |
|---|---|---|---|
| **dosage (mg)** | **theoretical cmax** | **effective cmax (post end of infusion)** | **conc. 3 h post end of infusion** |
| **2.5** | 0.71 | 0.06 - 0.14 | 0 |
| **5** | 1.43 | 0.32 - 0.44 | 0.09 - 0.16 |
| **10** | 2.86 | 0.8 - 2.0 | 0.46 - 1.4 |
| **20** | 5.71 | 2.3 - 4.1 | 1.7 - 3.3 |
| **40** | 11.43 | 5.9 - 6.5 | 4.4 - 6.1 |
| **60** | 17.14 | 8.9 - 15.1 | 7.7 - 11.08 |

Accordingly, in a preferred embodiment 0.2 to 10 mg of the agent is administered intravenously and the maximum concentration of the agent in the patient's plasma three hours after the administration is less than 2.5µg/ml. Preferably, 0.2 to 5 mg of the agent is administered intravenously and the maximum concentration of the agent in the patient's plasma three hours after the administration is less than 0.3µg/ml. Still more preferably, 0.5 to 3 mg of the agent is administered intravenously and the maximum concentration of the agent in the patient's plasma three hours after the administration is less than 0.1 µg/ml. These values are obtained after any administration and/or after the first and/or second administration of the agent.

The dose can also be calculated on the basis of the body surface area (BSA) of the subject. Body surface area (BSA) can be calculated according to any known method. Examples of BSA calculation methods are: the Mosteller formula of (BSA (m²) = ([Height(cm) x Weight(kg)]/ 3600)^{½} (Mosteller RD., N Engl J Med 1987 Oct 22;317(17):1098); the DuBois and DuBois formula of BSA (m²) = 0.20247 x Height(m)^{0.725} x Weight(kg)^{0.425} (DuBois D; DuBois EF., Arch Int Med 1916 17:863-71); the Haycock formula of BSA (m²) = 0.024265 x Height(cm)^{0.3964} x Weight(kg)^{0.5378} (Haycock G.B., et al., The Journal of Pediatrics 1978 93:1:62-66); the Gehan and George formula of BSA (m²) = 0.0235 x Height(cm)^{0.42246} x Weight(kg)^{0.51456} (Gehan EA, and George SL, Cancer Chemother Rep 1970 54:225-35); and the Boyd formula: BSA (m²) = 0.0003207 x Height(cm)^{0.3} x Weight(grams)^{(0.7285} - (0.0188 x LOG(grams)).

Accordingly, the agent can be administered to a subject in a dose from 0.1 to 5 mg/m² body surface area of the patient, preferably from 0.1 to 2.5 mg/m² and most preferably from 0.25 to 1.5 mg/m². Alternatively, the dose can be calculated based on the body weight of the subject, such that in a further aspect the agent is to be administered to a subject in a dose from 2 to 150 µg/kg, preferably 2 to 75 µg/kg, and most preferably from 5 to 45 µg/kg. As above, it is particularly preferred that these dosages are utilised when the agent is administered intravenously.

As indicated above, in one aspect the agent and/or methotrexate are administered subcutaneously. In general, as is known in the art, subcutaneous doses need to be larger than intravenous doses in order to achieve the equivalent therapeutic effect. The present inventors have demonstrated in monotherapy trials in rheumatoid arthritis patients with the antibody BT061 that the therapeutic effect achieved after 2 mg intravenous administration is approximately equivalent to that achieved after a 50 mg subcutaneous administration. These results are represented below in Table 2.

**Table 2 - the percentage of rheumatoid arthritis patients showing ACR improvements at week 7 after being treated with once weekly doses of BT061 (2 mg intravenous or 50 mg subcutaneous) for a total treatment period of six weeks. The results represent blinded data from 8 individuals, 2 receiving placebo and six receiving BT061.**

| **week 7** | **ACR20 (%)** | **ACR50 (%)** | **ACR70 (%)** |
|---|---|---|---|
| **2 mg i.v** | 50 | 25 | 12.5 |
| **50 mg s.c.** | 50 | 25 | 12.5 |

Accordingly, in a further preferred aspect the agent is administered to the patient subcutaneously or intramuscularly in a dose from 20 mg to 80 mg, and more preferably from 30 mg to 70 mg. Alternatively, the agent can be administered in a dosage from 8 to 50 mg/m² or from 0.2 to 1.5 mg/kg. In this aspect it is particularly preferred that the administration is at most about once every two weeks. It is noted that the aspects described in this paragraph can be combined with the other aspects and preferred features set out in this application.

The pharmaceutical composition or kit further comprises methotrexate (MTX). MTX treatment of RA is well known in the art and it is envisaged that MTX is to be administered in the dosages previously described. In particular, MTX is usually administered in a dose between 5 to 30 mg, preferably between 7.5 mg and 30 mg and most preferably between 10 to 25 mg. In some cases the dose will depend on the patient's pretreatment with MTX or tolerance to this drug.

In another aspect the method includes a further step of administering an additional therapeutic agent suitable for treating the rheumatic disease. Additional therapeutic agents comprise one or more of the following agents: a non-steroidal anti-inflammatory drug, an anti-inflammatory steroid, a gold compound, an anti-malarial drug, folic acid, cyclosporine, leflunomide, azathioprine, sulfasalazine, d-penicillamine, cyclophosphamide, mycophenoate, minocycline and chlorambucil. These additional therapeutic agents may be administered separately, simultaneously or sequentially with the agent capable of activating CD4+CD25+ regulatory T cells and the MTX.

As mentioned above the present inventors have surprisingly shown that the pharmaceutical composition and kit described herein are capable of treating rheumatic diseases. In particular, as shown below in Example 2 the treatment of rheumatoid arthritis results in a significant improvement in the disease. Accordingly, in a still further aspect where the disease is rheumatoid arthritis the treatment provides an improvement of the disease in the patient of at least ACR20, preferably at least ACR 50, and more preferably at least ACR 70, according to the American College of Rheumatology scoring system. In other words the treatment provides an at least 20%, preferably at least 50% and most preferably at least 70% improvement of the disease parameters according to the American College of Rheumatology (ACR) score of the patient.

Most preferably the treatment provides at least an ACR 70 response in the patient between 6 to 8 weeks after the start of treatment.

As can also be seen from Example 2 and the related Figures, the treatment described herein has the capacity to improve rheumatoid arthritis in a number of patients. Accordingly, the method of treatment is capable of treating rheumatoid arthritis by providing at least an improvement of the disease condition of ACR 20 to at least 20% of patients. Further, the method of treatment is capable of treating rheumatoid arthritis by providing at least an improvement of the disease condition of ACR 50, more preferably ACR 70, to at least 10% of patients.

The invention will now be described further with the following examples.

### EXAMPLE 1 In vitro proliferation assay with the antibody BT061 using freshly isolated CD4+CD25+ regulatory T cells

### Method

### Isolation of human CD4+CD25+ regulatory T cells

50 ml EDTA blood specimens were obtained from healthy control donors. Peripheral blood mononuclear cells (PBMCs), regulatory T cells (Tregs) and T helper cells as T responder cells (Tresps) were isolated from peripheral blood samples as previously described (Haas et al., J Immunol. 2007 Jul 15;179(2):1322-30).

### In vitro proliferation assays

Freshly isolated Tregs were pre-incubated for 48 hours with 1 µg/ml plate bound antibody (BT061), 1 µg/ml soluble BT061 or Medium.

Freshly isolated Tregs (2.5x10⁴, donor A) obtained from 2 donors (Exp. 1 and Exp. 2) were pre-incubated for 48 hours with either 1 µg/ml soluble or plate bound BT061. To achieve allogeneic stimulation the 2.5x10⁴ pre-incubated Tregs were then transferred to 1x10⁵ T cells as responder cells (Tresps) from a second donor (donor B) in the presence of 2x10⁵ T cell depleted and irradiated (30 Gray) PBMCs (donor A). After 4 days of stimulation 1 µCi [3H] thymidine per well was added and proliferation was measured after additional 16 hours.

### Results

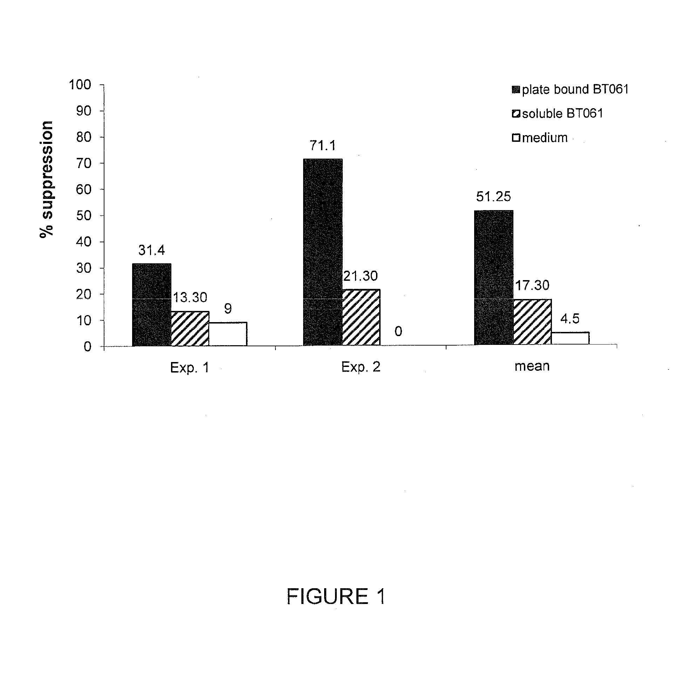

The percentage of Treg mediated inhibition of Tresp proliferation is shown in Figure 1 as the percent suppression of proliferation of Tresps incubated with PBMC in the absence of Tregs. Results are shown for the Tregs obtained from the two donors (Exp. 1 and Exp. 2). The dashed bars represent the results obtained with the Treg cells pre-incubated with soluble antibody, while the filled bars represent the results obtained with the Treg cells pre-incubated with plate bound antibody. As a control the suppressive activity of medium treated Tregs (open bars) is shown. Numbers above bars represent the percentage inhibition of Tresp proliferation.

As Figure 1 shows, Tregs pre-incubated with plate bound or soluble antibody were able to reduce average proliferative responses of allogeneic stimulated Tresps in contrast to Tregs incubated with medium alone. Further, the suppression obtained with plate-bound antibody was stronger compared to suppression obtained with soluble antibody.

Under physiological conditions in vivo BT061 as an IgG1 antibody is expected to bind to Fc receptors on Fc receptor expressing cells. This interaction would lead to recruitment of homogenously distributed BT061 (bound to CD4) into the local interaction site of target cells and Fc receptor expressing cells, leading to a cross-linking of BT061 and thus CD4. It is expected that interaction of BT061 with Fc receptor expressing cells confers a similar signal to Treg target cells as observed with the plate-bound antibody as both mechanisms recruit several target molecules (CD4) into close proximity on the cell surface.

### EXAMPLE 2 - Clinical trial in patients with rheumatoid arthritis

The ability of the pharmaceutical compositions and kits described herein to provide efficacious treatment of RA was demonstrated in patients suffering from RA.

The combination trial in which BT061 was studied in combination with MTX comprised a randomized placebo controlled double blind phase II study conducted in patients with moderate to severe RA. All patients had been taking stable doses of MTX for at least 3 months prior to the start of the trial, and these doses were continued in all patients the range of 15 to 20 mg per week during the course of the trial administered orally or intramuscularly.

The patients were divided into three groups. The patients in group I (14 patients) received a 0.5mg intravenous dose of BT061 and a dose of MTX in the range of 15 to 20 mg. The patients in group II (42 patients) received a 2.0 mg intravenous dose of BT061 and a dose of MTX in the range of 15 to 20 mg. The patients in group III (14 patients) received a dose of MTX in the range of 15 to 20 mg. The patients were dosed once a week over a period of eight weeks.

For intravenous administration the agent is to be infused in the forearm vein according to medically accepted procedures.

The treatment efficacy was evaluated weekly over the dosing period, and for a number of weeks after dosing was complete, by assessment of the ACR parameters (American Society of Rheumatology ACR homepage) and in particular studying the number of tender and swollen joints and following the levels of C-reactive protein (CRP) and the erythrocyte sedimentation rate (ESR). These parameters were also assessed before the trial to provide a "baseline" value at day 0.

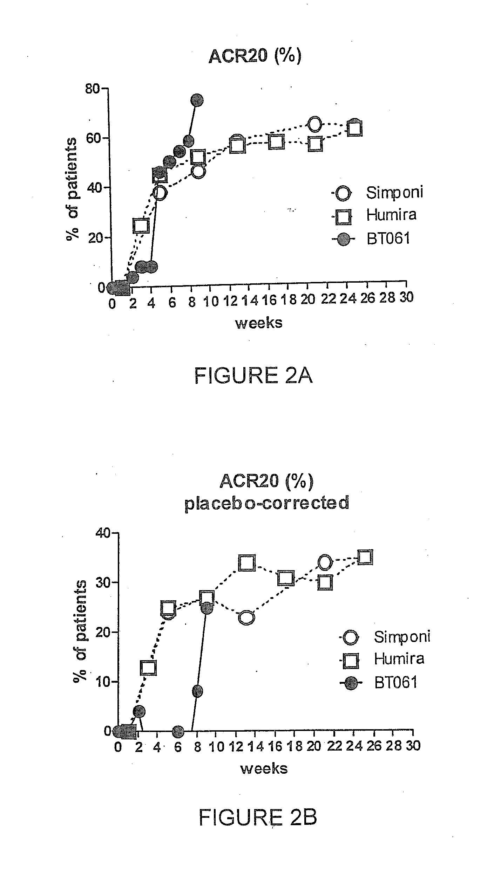

The results of the trial are shown in Figures 2 to 4 in which data obtained from patients in dose group II, receiving doses of 2mg BT061 + MTX, are compared with the most effective dose groups obtained in two published phase III trials involving the anti-TNF alpha antibodies, Humira (adalimumab) by Keystone et al., (Arthritis Rheum. 2004 May;50(5):1400-11 - trial DE019) and Simponi (golimumab) by Keystone et al., (Ann Rheum Dis. 2009 Jun;68(6):789-96 - Go-Forward trial) The most effective dose groups are shown for all of the studies.

It should be noted that the prior art results included for comparative purposes are phase III trials in which the doses of the pharmaceutical compositions have been optimized.

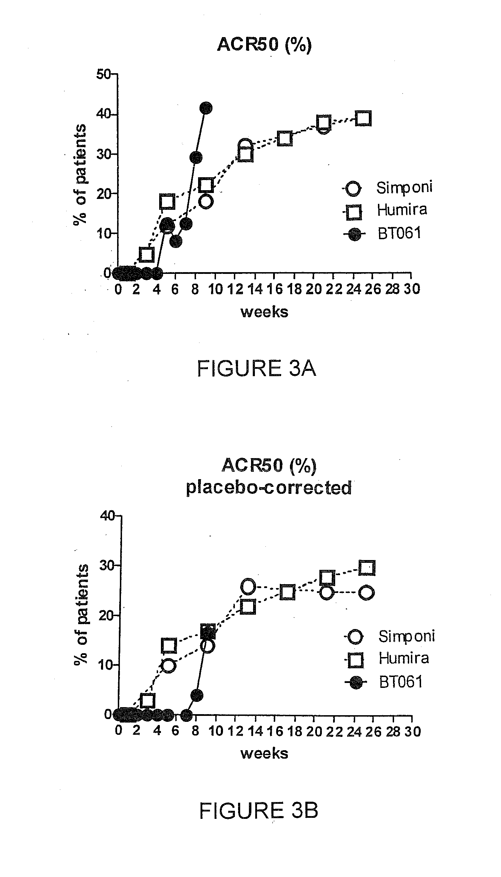

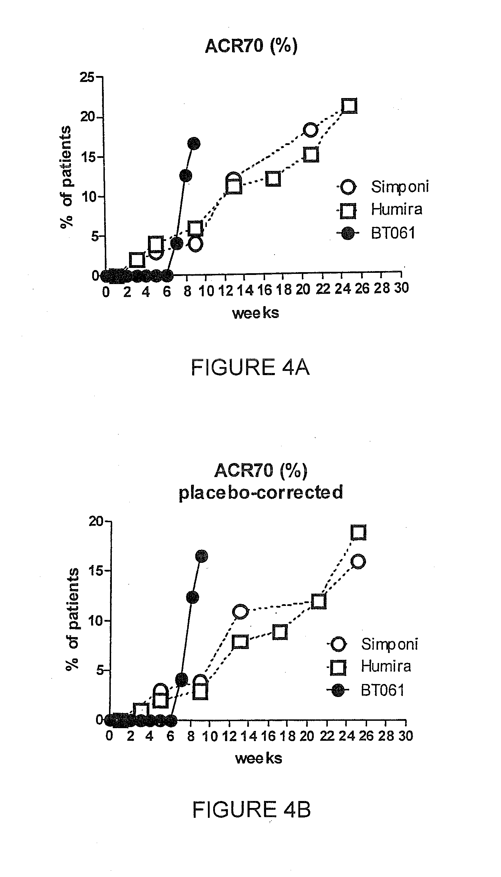

In particular, Figure 2 shows the percentage of patients from the 2mg dose group achieving an ACR20 score, while Figures 3 and 4 show the percentage of patients from the 2mg dose group achieving an ACR50 and an ACR 70 score, respectively.

As can be seen in the Figures, in the clinical trials with the therapeutic anti-TNF-alpha antibodies, peak therapeutic activity as measured by improvement of ACR scores usually requires several months. Generally, the percentage of patients showing an ACR20 response is maximal and reaches a plateau after 3 months. For ACR50 the plateau is reached after 4 months and for ACR70 the plateau is reached after 6 months.

However, the results of the combined therapy of the present invention show a number of differences. In particular, in the Figures showing the placebo-corrected results it can be seen that the onset of the therapeutic effect is delayed; the percentage of patients achieving an ACR20 score does not rise above 5% until week 8. However, after onset the therapeutic effect increases rapidly such that at week 9 the percentage of patients achieving ACR50 is comparable to that achieved in the phase III trials with the TNF-alpha antibodies, Humira and Simponi. The percentage of patients reaching ACR 20, ACR 50 and ACR70 increases rapidly between weeks 7 to 9 such that by week 9 the percentage of ACR20, ACR50 and ACR70 patients is approximately 25%, 18% and 17%, respectively. It is noted that this percentage of ACR70 patients is not reached in the trials with Humira and Simponi until 24 to 26 weeks after the start of treatment.

In addition the present inventors have noted that the number of adverse side effects seen with the combination therapy of BT061 and MTX is lower than that seen in the trials performed using BT061 alone, and therefore MTX has the capacity to reduce the side effects of therapeutic antibodies which activate CD4+CD25+ regulatory T cells.

These results demonstrate the efficacy and surprising advantages of the combination of an agent capable of activating CD4+CD25+ regulatory T cells and MTX of the present invention in the treatment of rheumatic disease.

## Claims

1. A kit comprising separately an agent capable of activating CD4+CD25+ regulatory T cells and methotrexate, wherein the agent is a humanized anti-CD4 antibody which comprises an H chain V domain and an L chain V domain, and wherein: (a) the H chain V domain comprises the sequences DCRMY, VISVKSENYGANYAESVRG and SYYRYDVGAWFAY, and the L chain V domain comprises the sequences RASKSVSTSGYSYIY, LASILES and QHSRELPWT; and/or (b) the H chain V domain has at least 80% sequence identity with SEQ ID No: 1 and the L chain V domain has at least 80% sequence identity with SEQ ID No: 2.

2. A kit according to claim 1 wherein the humanized anti-CD4 antibody comprises an H chain V domain defined by SEQ ID No: 1 and an L chain V domain defined by SEQ ID NO: 2.

3. An agent capable of activating CD4+CD25+ regulatory T cells and methotrexate as a combined preparation for simultaneous, separate or sequential use in medicine, wherein the agent is a humanized anti-CD4 antibody which comprises an H chain V domain and an L chain V domain, and wherein: (a) the H chain V domain comprises the sequences DCRMY, VISVKSENYGANYAESVRG and SYYRYDVGAWFAY, and the L chain V domain comprises the sequences RASKSVSTSGYSYIY, LASILES and QHSRELPWT; and/or (b) the H chain V domain has at least 80% sequence identity with SEQ ID No: 1 and the L chain V domain has at least 80% sequence identity with SEQ ID No: 2.

4. An agent capable of activating CD4+CD25+ regulatory T cells for use in the treatment of a rheumatic disease, wherein the agent is for simultaneous, separate or sequential use with methotrexate as a combination therapy, wherein the agent is a humanized anti-CD4 antibody which comprises an H chain V domain and an L chain V domain, and wherein: (a) the H chain V domain comprises the sequences DCRMY, VISVKSENYGANYAESVRG and SYYRYDVGAWFAY, and the L chain V domain comprises the sequences RASKSVSTSGYSYIY, LASILES and QHSRELPWT; and/or (b) the H chain V domain has at least 80% sequence identity with SEQ ID No: 1 and the L chain V domain has at least 80% sequence identity with SEQ ID No: 2.

5. An agent capable of activating CD4+CD25+ regulatory T cells for use in the treatment of a rheumatic disease in a patient, wherein the patient is undergoing methotrexate treatment, wherein the agent is a humanized anti-CD4 antibody which comprises an H chain V domain and an L chain V domain, and wherein: (a) the H chain V domain comprises the sequences DCRMY, VISVKSENYGANYAESVRG and SYYRYDVGAWFAY, and the L chain V domain comprises the sequences RASKSVSTSGYSYIY, LASILES and QHSRELPWT; and/or (b) the H chain V domain has at least 80% sequence identity with SEQ ID No: 1 and the L chain V domain has at least 80% sequence identity with SEQ ID No: 2.

6. A composition comprising methotrexate for use in the treatment of a rheumatic disease in a patient, wherein the patient is undergoing treatment with an agent capable of activating CD4+CD25+ regulatory T cells, wherein the agent is a humanized anti-CD4 antibody which comprises an H chain V domain and an L chain V domain, and wherein: (a) the H chain V domain comprises the sequences DCRMY, VISVKSENYGANYAESVRG and SYYRYDVGAWFAY, and the L chain V domain comprises the sequences RASKSVSTSGYSYIY, LASILES and QHSRELPWT; and/or (b) the H chain V domain has at least 80% sequence identity with SEQ ID No: 1 and the L chain V domain has at least 80% sequence identity with SEQ ID No: 2.

7. An agent capable of activating CD4+CD25+ regulatory T cells for use according to claim 4 in the treatment of a rheumatic disease in a patient who is a non-responder to treatment with a disease modifying anti-rheumatic drug (DMARD), preferably wherein the DMARD is methotrexate.

8. An agent or composition for use according to any one of claims 3 to 7 wherein the rheumatic disease is selected from rheumatoid arthritis, psoriatic arthritis, juvenile rheumatoid arthritis and ankylosing spondylitis, and preferably wherein the rheumatic disease is rheumatoid arthritis.

9. An agent or composition for use according to any one of claims 3 to 8 wherein the agent is suitable for administration as a single dose, as part of a plurality of doses, or once every week, every two weeks, every three weeks or every four weeks.

10. An agent or composition for use according to any one of claims 3 to 9 which is to be administered with an additional therapeutic agent suitable for treating the disease, selected from a non-steroidal anti-inflammatory drug, an anti-inflammatory steroid, a gold compound, an anti-malarial drug, folic acid, cyclosporine, leflunomide, azathioprine, sulfasalazine, d-penicillamine, cyclophosphamide, mycophenoate, minocycline and chlorambucil.

11. An agent or composition for use according to any one of claims 3 to 10 wherein the humanized anti-CD4 antibody comprises an H chain V domains defined by SEQ ID No: 1 and an L chain V domain defined by SEQ ID NO: 2.

12. A method comprising preparing a kit comprising the agent and the methotrexate according to claim 1 or claim 2.

## Patentansprüche

1. Kit umfassend ein separates Mittel, das in der Lage ist, regulatorische CD4+CD25+-T-Zellen zu aktivieren, und Methotrexat, wobei das Mittel ein humanisierter anti-CD4-Antikörper ist, der eine V-Domäne der H-Kette, und eine V-Domäne der L-Kette umfasst, und wobei:
(a) die V-Domäne der H-Kette die Sequenzen DCRMY, VISVKSENYGANYAESVRG und SYYRYDVGAWFAY umfasst, und die V-Domäne der L-Kette die Sequenzen RASKSVSTSGYSYIY, LASILES und QHSRELPWT umfasst; und/oder
(b) die V-Domäne der H-Kette eine wenigstens 80%-ige Sequenzidentität mit der Sequenz SEQ ID Nr. 1 aufweist, und die V-Domäne der L-Kette eine wenigstens 80%-ige Sequenzidentität mit der Sequenz SEQ ID Nr. 2 aufweist.

2. Kit nach Anspruch 1, wobei der humanisierte anti-CD4-Antikörper eine V-Domäne der H-Kette umfasst, die durch die Sequenz SEQ ID Nr. 1 definiert ist, sowie eine V-Domäne der L-Kette, die durch die Sequenz SEQ ID Nr. 2 definiert ist.

3. Mittel, das in der Lage ist, regulatorische CD4+CD25+-T-Zellen zu aktivieren, und Methotrexat als kombiniertes Präparat zur gleichzeitigen, separaten oder sequentiellen Verwendung in der Medizin, wobei das Mittel ein humanisierter anti-CD4-Antikörper ist, der eine V-Domäne der H-Kette und eine V-Domäne der L-Kette umfasst, und wobei
(a) die V-Domäne der H-Kette die Sequenzen DCRMY, VISVKSENYGANYAESVRG und SYYRYDVGAWFAY umfasst, und die V-Domäne der L-Kette die Sequenzen RASKSVSTSGYSYIY, LASILES und QHSRELPWT umfasst; und/oder
(b) die V-Domäne der H-Kette eine wenigstens 80%-ige Sequenzidentität mit der Sequenz SEQ ID Nr. 1 aufweist, und die V-Domäne der L-Kette eine wenigstens 80%-ige Sequenzidentität mit der Sequenz SEQ ID Nr. 2 aufweist.

4. Mittel, das in der Lage ist, regulatorische CD4+CD25+-T-Zellen zur Behandlung einer rheumatischen Erkrankung zu aktivieren, wobei das Mittel zur gleichzeitigen, separaten oder sequentiellen Verwendung mit Methotrexat als Kombinationstherapie dient, wobei das Mittel ein humanisierter anti-CD4-Antikörper ist, der eine V-Domäne der H-Kette und eine V-Domäne der L-Kette umfasst, und wobei
(a) die V-Domäne der H-Kette die Sequenzen DCRMY, VISVKSENYGANYAESVRG und SYYRYDVGAWFAY umfasst, und die V-Domäne der L-Kette die Sequenzen RASKSVSTSGYSYIY, LASILES und QHSRELPWT umfasst; und/oder
(b) die V-Domäne der H-Kette eine wenigstens 80%-ige Sequenzidentität mit der Sequenz SEQ ID Nr. 1 aufweist, und die V-Domäne der L-Kette eine wenigstens 80%-ige Sequenzidentität mit der Sequenz SEQ ID Nr. 2 aufweist.

5. Mittel, das in der Lage ist, regulatorische CD4+CD25+-T-Zellen zur Verwendung bei der Behandlung einer rheumatischen Erkrankung bei einem Patienten zu aktivieren, wobei der Patient einer Methotrexatbehandlung unterzogen wird, wobei das Mittel ein humanisierter anti-CD4-Antikörper ist, der eine V-Domäne der H-Kette und eine V-Domäne der L-Kette umfasst, und wobei
(a) die V-Domäne der H-Kette die Sequenzen DCRMY, VISVKSENYGANYAESVRG und SYYRYDVGAWFAY umfasst, und die V-Domäne der L-Kette die Sequenzen RASKSVSTSGYSYIY, LASILES und QHSRELPWT umfasst; und/oder
(b) die V-Domäne der H-Kette eine wenigstens 80%-ige Sequenzidentität mit der Sequenz SEQ ID Nr. 1 aufweist, und die V-Domäne der L-Kette eine wenigstens 80%-ige Sequenzidentität mit der Sequenz SEQ ID Nr. 2 aufweist.

6. Zusammensetzung umfassend Methotrexat zur Verwendung bei der Behandlung einer rheumatischen Erkrankung bei einem Patienten, wobei sich der Patient einer Behandlung mit einem Mittel unterzieht, das in der Lage ist, regulatorische CD4+CD25+-T-Zellen zu aktivieren, wobei das Mittel ein humanisierter anti-CD4-Antikörper ist, der eine V-Domäne der H-Kette und eine V-Domäne der L-Kette umfasst, und wobei
(a) die V-Domäne der H-Kette die Sequenzen DCRMY, VISVKSENYGANYAESVRG und SYYRYDVGAWFAY umfasst, und die V-Domäne der L-Kette die Sequenzen RASKSVSTSGYSYIY, LASILES und QHSRELPWT umfasst; und/oder
(b) die V-Domäne der H-Kette eine wenigstens 80%-ige Sequenzidentität mit der Sequenz SEQ ID Nr. 1 aufweist, und die V-Domäne der L-Kette eine wenigstens 80%-ige Sequenzidentität mit der Sequenz SEQ ID Nr. 2 aufweist.

7. Mittel, das in der Lage ist, regulatorische CD4+CD25+-T-Zellen zu aktivieren, zur Verwendung gemäß Anspruch 4 bei der Behandlung einer rheumatischen Erkrankung bei einem Patienten, der auf die Behandlung mit einem Disease-Modifying Anti-Rheumatic Drug (DMARD) nicht anspricht, wobei das DMARD bevorzugt Methotrexat ist.

8. Mittel oder Zusammensetzung zur Verwendung gemäß einem der Ansprüche 3 bis 7, wobei die rheumatische Erkrankung ausgewählt ist aus rheumatoider Arthritis, psoriatischer Arthritis, juveniler rheumatoider Arthritis und Spondilitis ankylosans, und wobei die rheumatoide Erkrankung bevorzugt rheumatoide Arthritis ist.

9. Mittel oder Zusammensetzung zur Verwendung gemäß einem der Ansprüche 3 bis 8, wobei das Mittel zur Verabreichung als Einzeldosis, als Teil mehrerer Dosen oder einmal pro Woche, alle zwei Wochen, alle drei Wochen oder alle vier Wochen geeignet ist.

10. Mittel oder Zusammensetzung zur Verwendung gemäß einem der Ansprüche 3 bis 9, welches/welche mit einem zusätzlichen therapeutischen Mittel verabreicht werden soll, das zur Behandlung der Erkrankung geeignet ist, ausgewählt aus einem nichtsteroidalen entzündungshemmenden Medikament, einem entzündungshemmenden Steroid, einer Goldverbindung, einem Medikament gegen Malaria, Folsäure, Cyclosporin, Leflunomid, Azathioprin, Sulfasalazin, d-Penicillamin, Cyclophsophamid, Mycophenoat, Minocyclin und Chlorambucil.

11. Mittel oder Zusammensetzung zur Verwendung nach einem der Ansprüche 3 bis 10, wobei der humanisierte anti-CD4-Antikörper eine V-Domäne der H-Kette, die durch die Sequenz SEQ ID Nr. 1 definiert ist, und eine V-Domäne der L-Kette, die durch die Sequenz SEQ ID Nr. 2 definiert ist, umfasst.

12. Verfahren umfassend die Herstellung eines Kits umfassend das Mittel und das Methotrexat gemäß Anspruch 1 oder Anspruch 2.

## Revendications

1. Un kit comprenant séparément un agent capable d'activer des lymphocytes T régulateurs CD4+CD25+ et du méthotrexate, dans lequel l'agent est un anticorps anti-CD4 humanisé qui comprend un domaine V de chaîne H, et un domaine V de chaîne L, et dans lequel : (a) le domaine V de chaîne H comprend les séquences DCRMY, VISVKSENYGANYAESVRG et SYYRYDVGAWFAY, et le domaine V de chaîne L comprend les séquences RASKVSTSGYSYIY, LASILES et QHSRELPWT ; et/ou (b) le domaine V de chaîne H présente une identité de séquence d'au moins 80 % avec SEQ ID No : 1 et le domaine V de chaîne L présente une identité de séquence d'au moins 80 % avec SEQ ID No : 2.

2. Le kit selon la revendication 1 dans lequel l'anticorps anti-CD4 humanisé comprend un domaine V de chaîne H défini par SEQ ID No : 1 et un domaine V de chaîne L défini par SEQ ID No : 2.

3. Un agent capable d'activer des lymphocytes T régulateurs CD4+CD25+ et du méthotrexate en tant qu'une préparation combinée pour une utilisation simultanée, séparée ou séquentielle en médecine, dans lequel l'agent est un anticorps anti-CD4 humanisé qui comprend un domaine V de chaîne H, et un domaine V de chaîne L, et dans lequel : (a) le domaine V de chaîne H comprend les séquences DCRMY, VISVKSENYGANYAESVRG et SYYRYDVGAWFAY, et le domaine V de chaîne L comprend les séquences RASKVSTSGYSYIY, LASILES et QHSRELPWT ; et/ou (b) le domaine V de chaîne H présente une identité de séquence d'au moins 80 % avec SEQ ID No : 1 et le domaine V de chaîne L présente une identité de séquence d'au moins 80 % avec SEQ ID No : 2.

4. Un agent capable d'activer des lymphocytes T régulateurs CD4+CD25+ pour une utilisation dans le traitement d'une maladie rhumatismale, dans lequel l'agent est pour une utilisation simultanée, séparée ou séquentielle avec du méthotrexate en tant que thérapie d'association, dans lequel l'agent est un anticorps anti-CD4 humanisé qui comprend un domaine V de chaîne H, et un domaine V de chaîne L, et dans lequel : (a) le domaine V de chaîne H comprend les séquences DCRMY, VISVKSENYGANYAESVRG et SYYRYDVGAWFAY, et le domaine V de chaîne L comprend les séquences RASKVSTSGYSYIY, LASILES et QHSRELPWT ; et/ou (b) le domaine V de chaîne H présente une identité de séquence d'au moins 80 % avec SEQ ID No : 1 et le domaine V de chaîne L présente une identité de séquence d'au moins 80 % avec SEQ ID No : 2.

5. Un agent capable d'activer des lymphocytes T régulateurs CD4+CD25+ pour une utilisation dans le traitement d'une maladie rhumatismale dans un patient, dans lequel le patient suit un traitement de méthotrexate, dans lequel l'agent est un anticorps anti-CD4 humanisé qui comprend un domaine V de chaîne H, et un domaine V de chaîne L, et dans lequel : (a) le domaine V de chaîne H comprend les séquences DCRMY, VISVKSENYGANYAESVRG et SYYRYDVGAWFAY, et le domaine V de chaîne L comprend les séquences RASKVSTSGYSYIY, LASILES et QHSRELPWT ; et/ou (b) le domaine V de chaîne H présente une identité de séquence d'au moins 80 % avec SEQ ID No : 1 et le domaine V de chaîne L présente une identité de séquence d'au moins 80 % avec SEQ ID No : 2.

6. Une composition comprenant du méthotrexate pour une utilisation dans le traitement d'une maladie rhumatismale dans un patient, dans laquelle le patient suit un traitement avec un agent capable d'activer des lymphocytes T régulateurs CD4+CD25+, dans laquelle l'agent est un anticorps anti-CD4 humanisé qui comprend un domaine V de chaîne H, et un domaine V de chaîne L, et dans lequel : (a) le domaine V de chaîne H comprend les séquences DCRMY, VISVKSENYGANYAESVRG et SYYRYDVGAWFAY, et le domaine V de chaîne L comprend les séquences RASKVSTSGYSYIY, LASILES et QHSRELPWT ; et/ou (b) le domaine V de chaîne H présente une identité de séquence d'au moins 80 % avec SEQ ID No : 1 et le domaine V de chaîne L présente une identité de séquence d'au moins 80 % avec SEQ ID No : 2.

7. Un agent capable d'activer des lymphocytes T régulateurs CD4+CD25+ pour une utilisation selon la revendication 4 dans le traitement d'une maladie rhumatismale dans un patient qui est non répondeur à un traitement avec un médicament antirhumatismal modificateur de la maladie (DMARD), de préférence dans lequel le DMARD est du méthotrexate.

8. Un agent ou une composition pour une utilisation selon l'une quelconque des revendications de 3 à 7 dans lequel la maladie rhumatismale est sélectionnée parmi la polyarthrite rhumatoïde, l'arthrite psoriasique, l'arthrite rhumatoïde juvénile et la spondylarthrite ankylosante, et de préférence dans lequel la maladie rhumatismale est la polyarthrite rhumatoïde.

9. Un agent ou une composition pour une utilisation selon l'une quelconque des revendications de 3 à 8 dans lequel l'agent est approprié pour être administré comme une dose unique, comme une partie d'une pluralité de doses, ou une fois par semaine, toutes les deux semaines, toutes les trois semaines ou toutes les quatre semaines.

10. Un agent ou une composition pour une utilisation selon l'une quelconque des revendications de 3 à 9 qui doit être administré avec un agent thérapeutique additionnel pour traiter la maladie, sélectionné parmi un médicament anti-inflammatoire non stéroïdien, un stéroïde anti-inflammatoire, un composé d'or, un médicament antipaludique, de l'acide folique, de la cyclosporine, du léflunomide, de l'azathioprine, de la sulfasalazine, de la D-pénicillamine, de la cyclophosphamide, du mycophénoate, de la minoclycline et du chlorambucil.

11. Un agent ou une composition pour une utilisation selon l'une quelconque des revendications de 3 à 10 dans lequel l'anticorps anti-CD4 humanisé comprend un domaine V de chaîne H défini par SEQ ID No : 1 et un domaine V de chaîne L défini par SEQ ID No : 2.

12. Un procédé comprenant la préparation d'un kit comprenant l'agent et le méthotrexate selon la revendication 1 ou la revendication 2.


### SEQUENCE LISTING

<110> Biotest AG
<120> Pharmaceutical Composition for Treating Disease
<130> 322248.WO/JND/CJS
<160> 16
<170> PatentIn version 3.3
<210> 1
   <211> 124
   <212> PRT
   <213> Artificial Sequence
<220>
   <223> V domain of H chain of humanized antibody hB-F5H37L
<400> 1
   Glu Glu Gln Leu Val Glu Ser Gly Gly Gly Leu Val Lys Pro Gly Gly
   1               5                   10                  15
   Ser Leu Arg Leu Ser Cys Ala Ala Ser Gly Phe Ser Phe Ser Asp Cys
               20                  25                  30
   Arg Met Tyr Trp Leu Arg Gln Ala Pro Gly Lys Gly Leu Glu Trp Ile
           35                  40                  45
   Gly Val Ile Ser Val Lys Ser Glu Asn Tyr Gly Ala Asn Tyr Ala Glu
       50                  55                  60
   Ser Val Arg Gly Arg Phe Thr Ile Ser Arg Asp Asp Ser Lys Asn Thr
   65                  70                  75                  80
   Val Tyr Leu Gln Met Asn Ser Leu Lys Thr Glu Asp Thr Ala Val Tyr
                   85                  90                  95
   Tyr Cys Ser Ala Ser Tyr Tyr Arg Tyr Asp Val Gly Ala Trp Phe Ala
               100                 105                 110
   Tyr Trp Gly Gln Gly Thr Leu Val Thr Val Ser Ser
           115                 120
<210> 2
   <211> 111
   <212> PRT
   <213> Artificial Sequence
<220>
   <223> V domain of K chain of humanized antibody hB-F5L4M
<400> 2
   Asp Ile Val Met Thr Gln Ser Pro Asp Ser Leu Ala Val Ser Leu Gly
   1               5                   10                  15
   Glu Arg Ala Thr Ile Asn Cys Arg Ala Ser Lys Ser Val Ser Thr Ser
               20                  25                  30
   Gly Tyr Ser Tyr Ile Tyr Trp Tyr Gln Gln Lys Pro Gly Gln Pro Pro
           35                  40                  45
   Lys Leu Leu Ile Tyr Leu Ala Ser Ile Leu Glu Ser Gly Val Pro Asp
       50                  55                  60
   Arg Phe Ser Gly Ser Gly Ser Gly Thr Asp Phe Thr Leu Thr Ile Ser
   65                  70                  75                  80
   Ser Leu Gln Ala Glu Asp Val Ala Val Tyr Tyr Cys Gln His Ser Arg
                   85                  90                  95
   Glu Leu Pro Trp Thr Phe Gly Gln Gly Thr Lys Val Glu Ile Lys
               100                 105                 110
<210> 3
   <211> 550
   <212> DNA
   <213> Artificial Sequence
<220>
   <223> Part of plasmid encoding V domain of H chain of humanized antibody hB-F5
<400> 3
   gaggagctcc agacaatgtc tgtctccttc ctcatcttcc tgcccgtgct gggcctccca     60
   tggggtcagt gtcagggaga tgccgtattc acagcagcat tcacagactg aggggtgttt    120
   cactttgctg tttccttttg tctccaggtg tcctgtcaga ggaacagctt gtggagtctg    180
   ggggaggctt ggtgaaaccc ggaggttctc tgaggctctc ctgtgcagcc tcgggtttca    240
   gtttcagtga ctgccggatg tactgggttc gccaggctcc agggaagggg ctggagtgga    300
   ttggtgtgat ttcagtcaaa tctgagaatt atggagcaaa ttatgcagag tctgtgaggg    360
   gcagattcac tatttcaaga gatgattcaa aaaacacggt ctatctgcag atgaacagct    420
   tgaagaccga agacactgcc gtttattatt gtagtgcctc ctattatagg tacgacgtgg    480
   gggcctggtt tgcttactgg ggccaaggga ctctggtcac tgtctcttca ggtaagaatg    540
   gccaagcttg                                                           550
<210> 4
   <211> 498
   <212> DNA
   <213> Artificial Sequence
<220>
   <223> Part of plasmid encoding V domain of K chain of humanized antibody hB-F5
<400> 4
   ggaggatcca attatctgct gacttataat actactagaa agcaaattta aatgacatat     60
   ttcaattata tctgagacag cgtgtataag tttatgtata atcattgtcc attcctgact    120
   acaggtgcct acggggacat cgtgatgacc cagtctccag actccctggc tgtgtctctg    180
   ggcgagaggg ccaccatcaa ctgcagggcc agcaaaagtg tcagtacatc tggctacagt    240
   tatatatatt ggtaccagca gaaaccagga cagcctccta agctgctcat ttaccttgca    300
   tccatcctag aatctggggt ccctgaccga ttcagtggca gcgggtctgg gacagatttc    360
   actctcacca tcagcagcct gcaggctgaa gatgtggcag tttattactg tcagcacagt    420
   agggaacttc cgtggacgtt cggccaaggg accaaggtgg aaatcaaacg tgagtagaat    480
   ttaaatttta agcttctt                                                  498
<210> 5
   <211> 124
   <212> PRT
   <213> Mus musculus
<400> 5
   Gln Glu Tyr Leu Val Glu Thr Gly Gly Gly Leu Val Arg Pro Gly Asn
   1               5                   10                  15
   Ser Leu Lys Leu Ser Cys Val Thr Ser Gly Phe Ser Phe Ser Asp Cys
               20                  25                  30
   Arg Met Tyr Trp Leu Arg Gln Pro Pro Gly Lys Gly Leu Glu Trp Ile
           35                  40                  45
   Gly Val Ile Ser Val Lys Ser Glu Asn Tyr Gly Ala Asn Tyr Ala Glu
       50                  55                  60
   Ser Val Arg Gly Arg Phe Thr Ile Ser Arg Asp Asp Ser Lys Ser Ser
   65                  70                  75                  80
   Val Tyr Leu Gln Met Ser Arg Leu Arg Glu Glu Asp Thr Ala Thr Tyr
                   85                  90                  95
   Tyr Cys Ser Ala Ser Tyr Tyr Arg Tyr Asp Val Gly Ala Trp Phe Ala
               100                 105                 110
   Tyr Trp Gly Gln Gly Thr Leu Val Thr Val Ser Ala
           115                 120
<210> 6
   <211> 111
   <212> PRT
   <213> Mus musculus
<400> 6
   Asp Ile Val Leu Thr Gln Ser Pro Ser Ser Leu Val Val Ser Leu Gly
   1               5                   10                  15
   Gln Arg Ala Thr Ile Ser Cys Arg Ala Ser Lys Ser Val Ser Thr Ser
               20                  25                  30
   Gly Tyr Ser Tyr Ile Tyr Trp Tyr Gln Gln Ile Pro Gly Gln Pro Pro
           35                  40                  45
   Lys Leu Leu Ile Tyr Leu Ala Ser Ile Leu Glu Ser Gly Val Pro Gly
       50                  55                  60
   Arg Phe Ser Gly Ser Gly Ser Gly Thr Asp Phe Thr Leu Asn Ile His
   65                  70                  75                  80
   Pro Val Glu Glu Glu Asp Ala Ala Thr Tyr Tyr Cys Gln His Ser Arg
                   85                  90                  95
   Glu Leu Pro Trp Thr Phe Gly Gly Gly Thr Lys Leu Glu Ile Lys
               100                 105                 110
<210> 7
   <211> 23
   <212> PRT
   <213> Homo sapiens
<400> 7
   Asp Ile Val Met Thr Gln Ser Pro Asp Ser Leu Ala Val Ser Leu Gly
   1               5                   10                  15
   Glu Arg Ala Thr Ile Asn Cys
               20
<210> 8
   <211> 15
   <212> PRT
   <213> Homo sapiens
<400> 8
   Trp Tyr Gln Gln Lys Pro Gly Gln Pro Pro Lys Leu Leu Ile Tyr
   1               5                   10                  15
<210> 9
   <211> 32
   <212> PRT
   <213> Homo sapiens
<400> 9
   Gly Val Pro Asp Arg Phe Ser Gly Ser Gly Ser Gly Thr Asp Phe Thr
   1               5                   10                  15
   Leu Thr Ile Ser Ser Leu Gln Ala Glu Asp Val Ala Val Tyr Tyr Cys
               20                  25                  30
<210> 10
   <211> 10
   <212> PRT
   <213> Homo sapiens
<400> 10
   Phe Gly Gln Gly Thr Lys Val Glu Ile Lys
   1               5                   10
<210> 11
   <211> 30
   <212> PRT
   <213> Homo sapiens
<400> 11
   Glu Val Gln Leu Val Glu Ser Gly Gly Gly Leu Val Lys Pro Gly Gly
   1               5                   10                  15
   Ser Leu Arg Leu Ser Cys Ala Ala Ser Gly Phe Thr Phe Ser
               20                  25                  30
<210> 12
   <211> 14
   <212> PRT
   <213> Homo sapiens
<400> 12
   Trp Val Arg Gln Ala Pro Gly Lys Gly Leu Glu Trp Val Gly
   1               5                   10
<210> 13
   <211> 32
   <212> PRT
   <213> Homo sapiens
<400> 13
   Arg Phe Thr Ile Ser Arg Asp Asp Ser Lys Asn Thr Leu Tyr Leu Gln
   1               5                   10                  15
   Met Asn Ser Leu Lys Thr Glu Asp Thr Ala Val Tyr Tyr Cys Thr Thr
               20                  25                  30
<210> 14
   <211> 11
   <212> PRT
   <213> Homo sapiens
<400> 14
   Trp Gly Gln Gly Thr Leu Val Thr Val Ser Ser
   1               5                   10
<210> 15
   <211> 124
   <212> PRT
   <213> Artificial Sequence
<220>
   <223> V domain of the H chain of humanized antibody hB-F5H37V
<400> 15
   Glu Glu Gln Leu Val Glu Ser Gly Gly Gly Leu Val Lys Pro Gly Gly
   1               5                   10                  15
   Ser Leu Arg Leu Ser Cys Ala Ala Ser Gly Phe Ser Phe Ser Asp Cys
               20                  25                  30
   Arg Met Tyr Trp Val Arg Gln Ala Pro Gly Lys Gly Leu Glu Trp Ile
           35                  40                  45
   Gly Val Ile Ser Val Lys Ser Glu Asn Tyr Gly Ala Asn Tyr Ala Glu
       50                  55                  60
   Ser Val Arg Gly Arg Phe Thr Ile Ser Arg Asp Asp Ser Lys Asn Thr
   65                  70                  75                  80
   Val Tyr Leu Gln Met Asn Ser Leu Lys Thr Glu Asp Thr Ala Val Tyr
                   85                  90                  95
   Tyr Cys Ser Ala Ser Tyr Tyr Arg Tyr Asp Val Gly Ala Trp Phe Ala
                       100                 105                 110
   Tyr Trp Gly Gln Gly Thr Leu Val Thr Val Ser Ser
           115                 120
<210> 16
   <211> 111
   <212> PRT
   <213> Artificial Sequence
<220>
   <223> V domain of K chain of humanized antibody hB-F5L4L
<400> 16
   Asp Ile Val Leu Thr Gln Ser Pro Asp Ser Leu Ala Val Ser Leu Gly
   1               5                   10                  15
   Glu Arg Ala Thr Ile Asn Cys Arg Ala Ser Lys Ser Val Ser Thr Ser
               20                  25                  30
   Gly Tyr Ser Tyr Ile Tyr Trp Tyr Gln Gln Lys Pro Gly Gln Pro Pro
           35                  40                  45
   Lys Leu Leu Ile Tyr Leu Ala Ser Ile Leu Glu Ser Gly Val Pro Asp
       50                  55                  60
   Arg Phe Ser Gly Ser Gly Ser Gly Thr Asp Phe Thr Leu Thr Ile Ser
   65                  70                  75                  80
   Ser Leu Gln Ala Glu Asp Val Ala Val Tyr Tyr Cys Gln His Ser Arg
                   85                  90                  95
   Glu Leu Pro Trp Thr Phe Gly Gln Gly Thr Lys Val Glu Ile Lys
               100                 105                 110